# EUROPEAN PATENT APPLICATION

(11) **EP 4 102 225 A1**
(43) Date of publication of application: **14.12.2022**
(21) Application number: 21792172.5
(22) Date of filing: 29.03.2021
(51) Int. Cl.: G01N 33/493, G01N 33/52, G01N 21/78

(54) **TEST PAPER FOR MEASURING MINERALS IN URINE**

(30) Priority: 23.04.2020 JP 2020076981
(71) Applicant: Yukashikado Inc., Tokyo 150-0001 (JP); Toyo Roshi Kaisha, Ltd., Tokyo 103-0023 (JP)
(72) Inventor: MINOBE, Shinya, Tokyo 150-0001 (JP); SAITO, Keiichiro, Tokyo 150-0001 (JP); ISHII,Yuya, Haga-gun, Tochigi 321-3325 (JP)
(74) Representative: Fleck, Hermann-Josef
(86) International application number: PCT/JP2021/013423
(87) International publication number: WO 2021/215204

(57) **Abstract**

Provided is a test paper for easily measuring the concentration of calcium in urine or the concentration of magnesium in urine. This test paper is for colorimetrically measuring the concentration of calcium in urine, the test paper including a coloring agent that colors in response to calcium ions on a substrate, a magnesium ion sealing agent for preventing coloration due to magnesium ions, a pH adjustment agent, and a sensitivity adjustment agent for adjusting the sensitivity of coloration within a range of concentrations of calcium ions in urine. According to another aspect, this test paper is for colorimetrically measuring the concentration of magnesium in urine, the test paper including a coloring agent that colors in response to magnesium ions on a substrate, a calcium ion sealing agent for preventing coloration due to calcium ions, a pH adjustment agent, and a sensitivity adjustment agent for adjusting the sensitivity of coloration within a range of concentrations of magnesium ions in urine.

## Description

### [Technical Field]

The present invention relates to test papers for measuring the concentration of minerals in urine, particularly the concentration of calcium in urine or the concentration of magnesium in urine.

### [Background Art]

A urine test paper is a simple and quick way to test variations in substances contained in urine by colorimetric measurements. The urine test paper allows the immediate judgment of the health condition of the subject by grasping the variation in the color tone of the component to be tested from the standard color for healthy subjects. Therefore, the urine test paper is widely used for the purpose of mass examinations or health management at home.

Currently, items tested using urine test papers include pH, specific gravity, protein, glucose, occult blood reaction, ketone bodies, bilirubin, urobilinogen, nitrite, leukocyte reaction, creatinine and albumin. In each item, the urine test paper shows a unique color development, and it is possible to judge whether it is normal or abnormal by comparing the color tone and the concentration of the standard color development and observing the color development state of the subject's urine. The judgment can be done by visually comparing with the color tone chart attached to the test paper container, or by reading using an automatic device.

As a urine test paper, for example, a test paper in which test sites corresponding to a plurality of test items are provided on a strip-shaped support as disclosed in Patent Document 1 is common. That is, the urine test paper is prepared by cutting into squares a base material such as filter paper impregnated with various reagents corresponding to the above-mentioned various analysis items on a strip-shaped support made of plastic, nonwoven fabric, or paper, and is arranged on the support so as to correspond to a plurality of analysis items. The method of using the urine test paper is the so-called dip-and-read method, in which the urine test paper is immersed in the urine to be tested in a cup or the like and then pulled up, and in which the analysis result is judged by comparing with color samples prepared in advance when the color reaction of the detection site on the urine test paper is read visually or mechanically.

In addition to the urine test as described above, a so-called blood test for measuring the concentration of various components in serum by collecting blood is indispensable for grasping the health condition from various aspects.

Ion concentrations of sodium, potassium, calcium, magnesium, etc. as various inorganic ions in the blood are controlled within an extremely narrow range in a normal healthy person. However, low values and high values from the normal range often reflect the effects of changing health conditions. Examples include hypocalcemia and hypercalcemia, in which blood calcium levels continue to fluctuate from normal values.

Calcium is one of the most important minerals in the human body and is essential for neurotransmitter exocytosis, not only for maintaining bone mineral density. Calcium is also involved in muscle cell contraction and is essential as a depolarizing mineral in the heart. Thus, maintaining calcium is essential for maintaining normal functioning in the body. Calcium is ingested through meals and absorbed into the body mainly through the small intestinal wall. When the mucosa of the small intestinal wall is damaged, it causes a syndrome called (primary) malabsorption syndrome (Non-Patent Document 1), which presents various symptoms such as anemia, abdominal distension, edema, and malaise.

Also, if the state of malabsorption of calcium continues, osteoporosis and other metabolic bone diseases are caused, so calcium should be taken on daily basis. It is preferable to monitor the absorption of calcium along with the intake, and it is possible to know the degree of absorption into the body by measuring the amount of ingested calcium excreted in the urine.

As a method for measuring the concentration of calcium in urine, a colorimetric method (Arsenazo III) using a sample collected for 24 hours is used, but there is a problem that the reagent contains arsenic. Also, when trying to measure the concentration of calcium in urine by the test paper method, there is a problem that the judgment result may be a false negative due to interference by the presence of other inorganic ions and organic substances. At present, the actual situation is that there is no easy method for measuring the concentration of calcium in urine on the spot. Especially, if it were possible to measure the concentration of calcium in urine using a test paper, it would be possible to easily know whether the intake and absorption of calcium are within an appropriate range. Therefore, although it would be extremely useful, such a test paper and a method for evaluating nutritional status using this test paper do not exist.

Also, regarding the concentration of magnesium in urine, if the concentration of magnesium in blood is high (low), the concentration of magnesium excreted in urine is also high (low). Therefore, measurement of the concentration of magnesium in urine can be used indirectly. At present, the standard test method for measuring the concentration of magnesium in urine is the xylidine blue method using 1 ml of collected urine, but no test method using the urine test paper has been developed. In the body, magnesium is widely distributed in bones and the like, and when bone resorption by osteo-clasts is accelerated in the above bone diseases, magnesium is released into the blood and the concentration of magnesium ions in urine also shows high values. Therefore, a method for easily measuring the concentration of magnesium ions in urine is desired.

### [Prior Art]

### [Patent Document]

[Patent Document 1] JP 1997-105747 A

### [Non-patent Document]

[Non-patent Document 1] Yoshihiro Fukuda, "Jomyaku Keicho Eiyo" , Vol.27, No.1, pp 5-17, 2012.

### [Outline of the Invention]

### [Problems to be Solved by the Invention]

In view of the above situation, an object of the present invention is to provide a test paper for easily measuring the concentration of calcium in urine or the concentration of magnesium in urine.

### [Means to Solve the Objects]

The above problems are solved by using the means shown below.

A test paper according to a first aspect of the present invention is for colorimetrically measuring the concentration of calcium in urine, and includes a coloring agent that colors in response to calcium ions on a substrate, a magnesium ion sealing agent for preventing coloration due to magnesium ions, a pH adjustment agent, and a sensitivity adjustment agent for adjusting the sensitivity of coloration within a range of concentrations of calcium ions in urine.

According to a configuration of the test paper according to the first aspect, the concentration of calcium in urine can be easily measured by a colorimetric method.

A test paper according to a second aspect of the present invention is for colorimetrically measuring the concentration of magnesium in urine, and includes a coloring agent that colors in response to magnesium ions on a substrate, a calcium ion sealing agent for preventing coloration due to calcium ions, a pH adjustment agent, and a sensitivity adjustment agent for adjusting the sensitivity of coloration within a range of concentrations of magnesium ions in urine.

According to a configuration of the test paper according to the second aspect, the concentration of magnesium in urine can be easily measured by a colorimetric method.

Cresolphthalein complexone can be suitably used as the coloring agent in the test paper according to the first aspect. Cresolphthalein complexone can be used as the coloring agent because it reacts with calcium and magnesium to form a reddish-purple complex under basic conditions.

Also, 8-Hydroxyquinoline-5-sulfonic acid can be suitably used as the magnesium ion sealing agent in the test paper according to the first aspect. 8-Hydroxyquinoline-5-sulfonic acid is used to remove magnesium because it selectively reacts with magnesium.

Cresolphthalein complexone can be suitably used as the coloring agent in the test paper according to the second aspect. Also, G-EDTA can be preferably used as the calcium ion sealing agent in the test paper according to the second aspect.

A test paper according to a third aspect of the present invention includes both the test paper according to the first aspect and the test paper according to the second aspect.

A mixture of sodium carbonate and sodium bicarbonate can be used as the pH adjustment agent in the test paper according to the first or second aspect. Also, sodium citrate or sodium pyrophosphate can be used as the sensitivity adjustment agent in the test paper according to the first or second aspect. For example, cresolphthalein complexone, which can be used as a coloring agent, is originally a highly sensitive reagent, and since the coloration reaction saturates at low concentration, one can adjust the sensitivity using the complex formation reaction of sodium citrate, calcium, and magnesium so that the coloration changes within a predetermined concentration range.

### [Effects of the Invention]

According to the test paper of the present invention, there is an effect that the concentration of calcium in urine and the concentration of magnesium in urine can be easily measured.

### [Brief Description of the Drawings]

[Fig. 1] Fig. 1 shows a liquid preparation flow chart of test papers for measuring the concentration of calcium in urine in Embodiment 1.
[Fig. 2] Fig. 2 shows a liquid preparation flow chart of test papers for measuring the concentration of calcium in urine in Embodiment 2.
[Fig. 3] Fig. 3 shows a fabrication flow chart of test papers for measuring the concentration of calcium in urine.
[Fig. 4] Fig. 4 shows a liquid preparation flow chart of urine test papers for measuring the concentration of magnesium in urine in Embodiment 4.
[Fig. 5] Fig. 5 shows a liquid preparation flow chart of urine test papers for measuring the concentration of magnesium in urine in Embodiment 5.
[Fig. 6] Fig. 6 shows a fabrication flow chart of test papers for measuring the concentration of magnesium in urine.

### [Best Mode for Carrying Out the Invention]

The present invention relates to a test paper for measuring the concentration of calcium and/or the concentration of magnesium contained in urine. In the test paper, measurement sites where the color development intensity increases in proportion to the concentration of calcium in urine and measurement sites where the color development intensity increases in proportion to the concentration of magnesium in urine are simultaneously provided on a support. With such a configuration, the detection of each ion concentration is not hindered by the presence of other ions, and both ion concentrations can be measured simultaneously in one measurement.

Some supports that can be used in the present invention include paper, plastic, or non-woven fabric, which are cut into long strips, and the surface of the supports are configured by laminating other measurement sites containing various coloring reagents corresponding to test items for measuring. A plurality of measurement sites are arranged side by side on the support in order to perform measurements of two or more types as measurement items in one measurement at the same time. The substrate is particularly preferably paper. As the base material, it is preferable to use a case where the base material has water resistance enough to maintain its shape until it is immersed in urine in a urine test until the coloring reaction is completed, and is decomposed in the environment by being discarded after measuring.

The test paper of the present invention has measurement sites where the coloring intensity increases in proportion to the concentration of calcium in urine and measurement sites where the coloring intensity increases in proportion to the concentration of magnesium in urine; these sites are provided together on the above support.

Here, it is known that the normal concentration of calcium in serum is controlled in a relatively narrow range of 88 to 103 ppm, which is generally diagnosed as hypercalcemia when the range is 104 ppm or more and hypocalcemia when the range is less than 88 ppm. On the other hand, it is known that the concentration of calcium contained in urine widely ranges from 20 to 400 ppm.

The test paper according to the first aspect of the present invention is characterized in that the color development intensity changes continuously at the concentration of calcium in urine in the range of at least 20 to 400 ppm, whereby it is possible to measure conversely the concentration of calcium contained in urine from the color development intensity. In the test paper according to the first aspect of the present invention, the measurement range of the concentration of calcium in urine is at least in the range of 20 to 400 ppm, more preferably in the range of 10 to 600 ppm, and particularly preferably in the range of 0 to 1000 ppm.

Also, the concentration of magnesium in serum is maintained in the range of 18 to 26 ppm, while the concentration of magnesium in urine is usually in the wide range of 18 to 215 ppm. Hypermagnesemia is the condition in which the concentration of magnesium in serum exceeds 26 ppm, and the main cause is renal failure. Hypomagnesemia is the condition in which the concentration of magnesium in serum is less than 18 ppm, and is suspected to be due to insufficient magnesium intake, insufficient absorption, or increased urine output due to hypercalcemia.

The test paper according to the second aspect of the present invention is characterized in that the color development intensity changes continuously at the concentration of magnesium in urine in the range of at least 18 to 215 ppm, whereby it is possible to measure conversely the concentration of magnesium contained in urine from the color development intensity. In the test paper according to the second aspect of the present invention, the measurement range of the concentration of magnesium in urine is at least in the range of 18 to 215 ppm, more preferably in the range of 10 to 300 ppm, and particularly preferably in the range of 0 to 500 ppm.

The concentration of calcium in urine and the concentration of magnesium in urine occupy an important position in maintaining nutritional status. The test paper according to the first aspect of the present invention contains a coloring reagent that reacts with calcium ions in urine, and includes a magnesium ion sealing agent for sealing magnesium ions simultaneously contained in urine, a pH adjustment agent for allowing the color development reaction to occur within an appropriate pH range, and a sensitivity adjustment agent for adjusting the color development reaction within a range of concentrations of calcium ions in urine.

According to the test paper according to the first aspect of the present invention, it is possible to accurately measure the concentration of calcium in urine without being disturbed by the presence of inorganic ions other than calcium and other organic substances. Inorganic ions other than calcium contained in urine include magnesium, sodium, potassium, and ammonium ions, which combine with chloride ions, sulfate ions, phosphate ions, etc., and form a chelate with citric acid, oxalic acid, etc.

In the present invention, the concentration of calcium in urine is detected by utilizing a color development reaction upon reaction with a chelating reagent. Examples of such coloring agents include cresolphthalein complexone (3,3'-Bis[N,N-bis(carboxymethyl)aminomethyl]-o-cresolphthalein). Cresolphthalein complexone is characterized by strong color development in the visible region having an absorption maximum near 570 nm by binding with calcium or magnesium ions. In order to use this reagent for measuring the concentration of calcium in urine, it is necessary to seal a reaction with magnesium cocontained in urine. Examples of reagents that selectively bind magnesium ions in solution include 8-hydroxyquinoline-5-sulfonic acid.

The most preferred example of the coloring agent that can be used in the test paper of the present invention is cresolphthalein complexone, and the most preferred example of the magnesium ion sealing agent is 8-hydroxyquinoline-5-sulfonic acid.

Thymolphthalein complexone and eriochrome black T (sodium 1-(1-hydroxy-2-naphthylazo)-6-nitronaphthol-4-sulfonate) can also be used as an example of other coloring agents.

In addition, there is a coloring agent that does not react with magnesium in a strongly acidic region but selectively reacts with calcium ions to develop color. When such a coloring agent is used in the strongly acidic region, it can be used alone without requiring the magnesium ion sealing agent. However, in this case, there is a problem in storage stability because the paper of the support deteriorates in strongly acidic conditions. Therefore, the pH at which the color development reaction is performed is preferably neutral to alkaline.

The test paper according to the second aspect of the present invention contains a coloring reagent that reacts with magnesium ions in urine, and includes a calcium ion sealing agent for sealing calcium ions simultaneously contained in urine, a pH adjustment agent for allowing the color development reaction to occur within an appropriate pH range, and a sensitivity adjustment agent for adjusting the color development reaction within a range of concentrations of magnesium ions in urine.

As a reagent that reacts with magnesium ions to develop color, cresolphthalein complexone is widely used, but in addition to this, thiazole yellow, brilliant yellow, etc. may also be used, but in the present invention, cresolphthalein complexones are most preferably used.

Since cresolphthalein complexone reacts with not only magnesium ions but also calcium ions to develop a color, it is preferable to also use a calcium ion sealing agent. As the calcium ion sealing agent that can be used for such purposes, it can be preferably used that a chelating reagent such as G-EDTA (ethyleneglycol-bis(2-aminoethylethylether-N,N,N',N'-tetraacetic acid). In this case, since G-EDTA selectively binds with calcium ions at pH 10 alkalinity, it can be used as a calcium ion sealing agent, and it can be preferably used because magnesium ions remaining in the solution react with cresolphthalein complexone to develop a strong color.

Other calcium ion sealing agents include calcein (3,3'-bis[N,N-di(carboxymethyl)aminomethyl]fluorescein). Calcein selectively binds to calcium ions to produce pale red fluorescence, but does not develop color in the visible light region, so it can be used as a sealing agent for calcium ions in urine. However, in this case, it is possible to measure the concentration of calcium ions by irradiating with an ultraviolet lamp or the like, but it is not suitable for visual evaluation so as to interfere with the color development of magnesium, and is not necessarily suitable for the purpose of the present invention. Conversely, however, one type of test paper can measure both calcium ions and magnesium ions at the same time by measuring fluorescence and visible light.

According to the first and second aspects of the present invention, the test paper contains a pH adjustment agent for carrying out the color development reaction on the moderately alkaline side. As the pH adjustment agent that can be used in this case, an alkaline buffer having buffering properties on the alkaline side is preferable. For example, a mixture of sodium carbonate and sodium bicarbonate, a mixture of sodium bicarbonate and sodium hydroxide, a mixture of disodium hydrogen phosphate and sodium hydroxide, a mixture of glycine and sodium hydroxide, and the like. In particular, the mixture of sodium carbonate and sodium bicarbonate can be used particularly preferably as pH adjustment agents in the test papers of the present invention.

Coloring agents such as cresolphthalein complexone that can be used as a coloring agent in the present invention react with a trace amount of calcium ions or magnesium ions to develop a color. Therefore, if this is directly reacted with calcium ions and magnesium ions in urine, the high concentration of these ions saturates the color development reaction and cannot be used for measuring the concentration. Therefore, for the binding reaction between calcium ions or magnesium ions in urine and the coloring agent it is preferable to use a sensitivity adjustment agent that moderately adjusts the concentration of calcium ions that react with the coloring agent so as to cover a measurement range of the concentration of calcium in urine of at least 20 to 400 ppm, more preferably 10 to 600 ppm, and particularly preferably 0 to 1000 ppm.

Similarly, it is preferable to use a sensitivity adjustment agent that moderately adjusts the concentration of magnesium ions that react with the coloring agent so as to cover a measurement range of the concentration of magnesium in urine of at least 18 to 215 ppm, more preferably 10 to 300 ppm, and particularly preferably 0 to 500 ppm.

Various chelating agents can be used as sensitivity adjustment agents that can be used for such purposes. For example, EDTA, sodium citrate, glycine, DHEG (N,N-Bis(2-hydroxyethyl)glycine) and the like can be used, and among them sodium citrate is most preferably used.

Next, a method for evaluating the nutritional status of a subject from measurement of the concentration of calcium in urine and the concentration of magnesium in urine using the test paper of the present invention will be described.

The test paper of the present invention can be used to evaluate the nutritional status of a subject by a single measurement of the concentration of calcium in urine, but preferably, measurements are performed multiple times over several days, and the nutritional status of the subject is preferably evaluated appropriately based on the results. The concentration of calcium in urine increases or decreases depending on the intake of calcium from food or the like. Since the amount of calcium absorbed from the small intestine is controlled by the actions of parathyroid hormone and the like, calcium that has not been absorbed into the bones is excreted in urine. When the amount of calcium intake is low, the concentration of calcium excreted in urine is also low, so it is possible to judge whether calcium supplementation is necessary or not. It is beneficial to know of a low value of the concentration of calcium in urine. Furthermore, a low value of the concentration of calcium in urine throughout a day may also suggest the possibility of hypocalciuric hypercalcemia, e.g., low concentration of calcium in urine and high concentration of calcium in blood. Therefore, it is significant for maintaining health to know of a low value of the concentration of calcium in urine.

On the other hand, a high value of the concentration of calcium in urine due to excessive intake of calcium increases suspicion of hypercalcemia, so special attention is required.

Also, when the concentration of magnesium in urine is a low value throughout a day, insufficient intake of magnesium is suspected, which serves as an opportunity to encourage appropriate intake. Conversely, when the concentration of magnesium in urine is a high value, the possibility of renal failure or hypercalcemia is detected, which is an important index for maintaining health. In this way, measurement of the concentration of calcium or magnesium in urine throughout a day is an extremely important index for maintaining nutritional status. So, it can be said that the test paper for measuring the concentration of calcium in urine and the test paper for measuring the concentration of magnesium in urine are extremely useful.

Embodiments of the present invention will be described in detail below with reference to the drawings. The present invention is not limited to the following embodiments and examples shown in the figures, and the present invention can be variously changed in design.

### [Embodiment 1]

### (Liquid preparation and procedure 1 of test papers for measuring the concentration of calcium in urine)

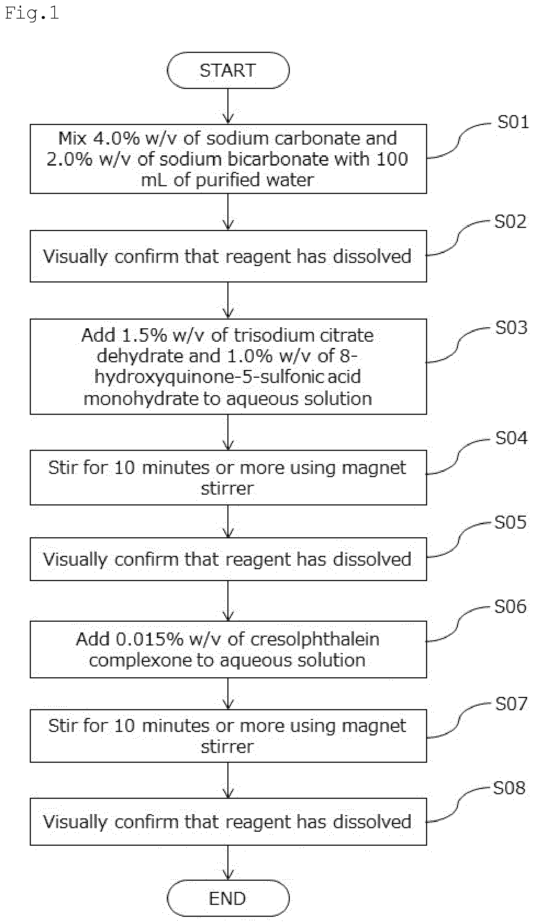

Fig. 1 shows a liquid preparation flow chart of test papers for measuring the concentration of calcium in urine in Embodiment 1. As shown in Fig. 1, first, 4.0% w/v of sodium carbonate and 2.0% w/v of sodium bicarbonate were mixed with 100 mL of purified water (step S01), and it was visually confirmed that the reagent had dissolved (step S02). Next, 1.5% w/v of trisodium citrate dehydrate and 1.0% w/v of 8-hydroxyquinone-5-sulfonic acid monohydrate were added to this aqueous solution (step S03), and after stirring for 10 minutes or more using a magnet stirrer (SRS311HA manufactured by Toyo Seisakusho Co., Ltd.) (step S04), it was visually confirmed that the reagent had dissolved (step S05). Finally, 0.015% w/v cresolphthalein complexone was added to this aqueous solution (step S06), and after stirring for 10 minutes or more using the magnetic stirrer (step S07), it was visually confirmed that the reagent had dissolved (step S08).

### [Embodiment 2]

### (Liquid preparation and procedure 2 of test papers for measuring the concentration of calcium in urine)

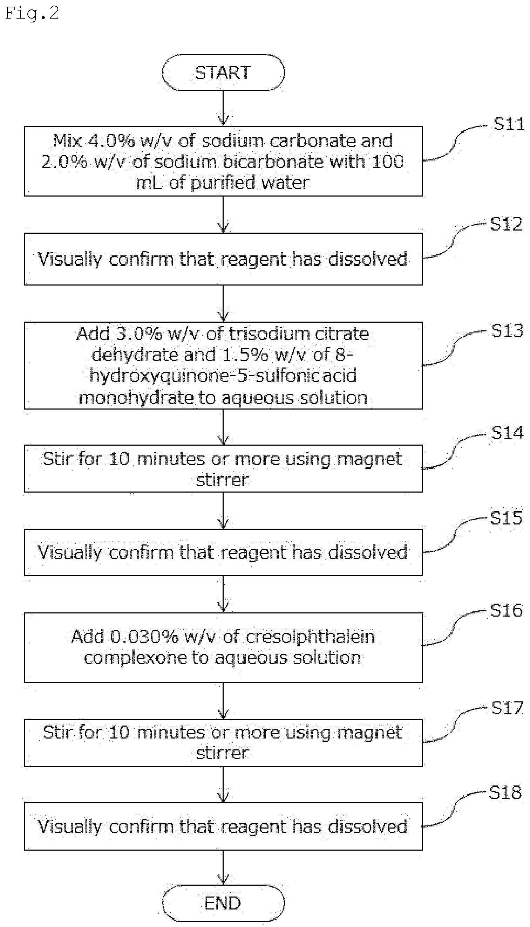

Fig. 2 shows a liquid preparation flow chart of test papers for measuring the concentration of calcium in urine in Embodiment 2. As shown in Fig. 2, first, 4.0% w/v of sodium carbonate and 2.0% w/v of sodium bicarbonate were mixed with 100 mL of purified water (step S11), and it was visually confirmed that the reagent had dissolved (step S12). Next, 3.0% w/v of trisodium citrate dehydrate and 1.5% w/v of 8-hydroxyquinone-5-sulfonic acid monohydrate were added to this aqueous solution (step S13), and after stirring for 10 minutes or more using a magnet stirrer (SRS311HA manufactured by Toyo Seisakusho Co., Ltd.) (step S14), it was visually confirmed that the reagent had dissolved (step S15). Finally, 0.030% w/v cresolphthalein complexone was added to this aqueous solution (step S16), and after stirring for 10 minutes or more using the magnetic stirrer (step S17), it was visually confirmed that the reagent had dissolved (step S18).

Cresolphthalein complexone is used as a coloring agent because it reacts with calcium and magnesium under basic conditions to form a reddish-purple complex. When cresolphthalein complexone is used as a coloring agent, because it reacts with calcium and magnesium under basic conditions, a carbonate buffer is used. When using cresolphthalein complexone, since cresolphthalein complexone is a highly sensitive reagent and coloration saturates at low concentration, the sensitivity is adjusted using the complex formation reaction of sodium citrate, calcium, and magnesium so that the coloration changes within a target concentration range.

### [Embodiment 3]

### (Method for preparing test papers for measuring the concentration of calcium in urine)

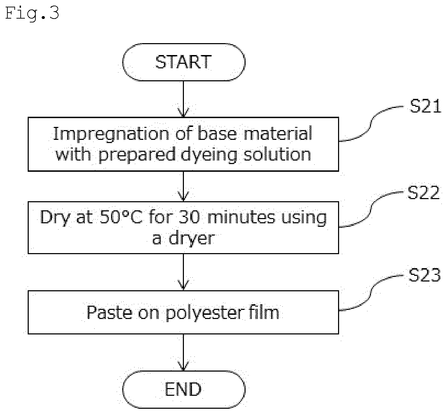

Fig. 3 shows a fabrication flow chart of test papers for measuring the concentration of calcium in urine. As shown in Fig. 3, the base material (quantitative filter paper No. 3 manufactured by Toyo Roshi Kaisha, Ltd.) was impregnated with the prepared dyeing solution (step S21), and was dried at 50 °C, for 30 minutes using a dryer (DRK432DC manufactured by Toyo Seisakusho Co., Ltd.) (step S22). This was pasted on a polyester film of 85 mm x 5 mm (step S23), and the test paper was completed.

### [Embodiment 4]

### (Liquid preparation and procedure 1 of test papers for measuring the concentration of magnesium in urine)

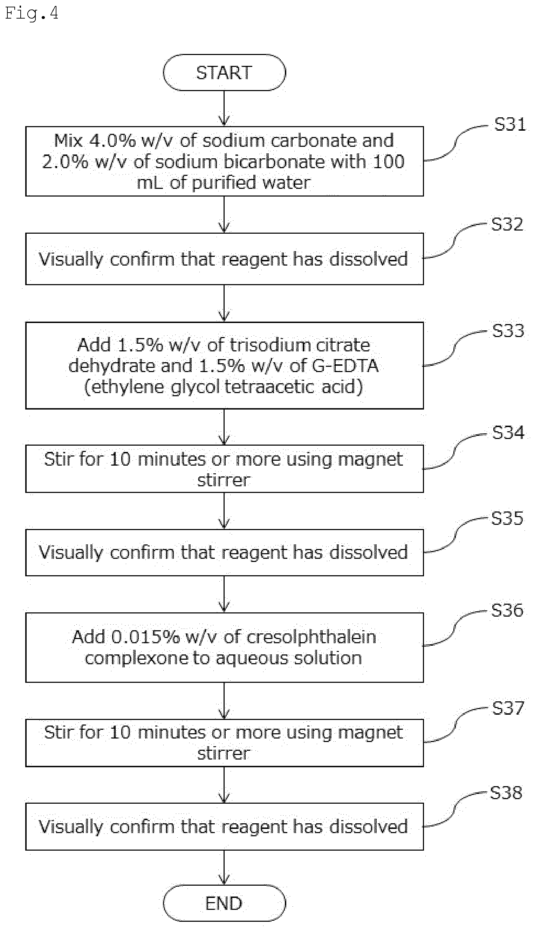

Fig. 4 shows a liquid preparation flow chart of test papers for measuring the concentration of magnesium in urine in Embodiment 4. As shown in Fig. 4, first, 4.0% w/v of sodium carbonate and 2.0% w/v of sodium bicarbonate were mixed with 100 mL of purified water (step S31), and it was visually confirmed that the reagent had dissolved (step S32). Next, 1.5% w/v of trisodium citrate dehydrate and 1.5% w/v of G-EDTA (ethylene glycol tetraacetic acid) were added to this aqueous solution (step S33), and after stirring for 10 minutes or more using a magnet stirrer (for example, SRS311HA manufactured by Toyo Seisakusho Co., Ltd.) (step S34), it was visually confirmed that the reagent had dissolved (step S35). Finally, 0.015% w/v cresolphthalein complexone was added to this aqueous solution (step S36), and after stirring for 10 minutes or more using the magnetic stirrer (step S37), it was visually confirmed that the reagent had dissolved (step S38).

### [Embodiment 5]

### (Liquid preparation and procedure 2 of test papers for measuring the concentration of magnesium in urine)

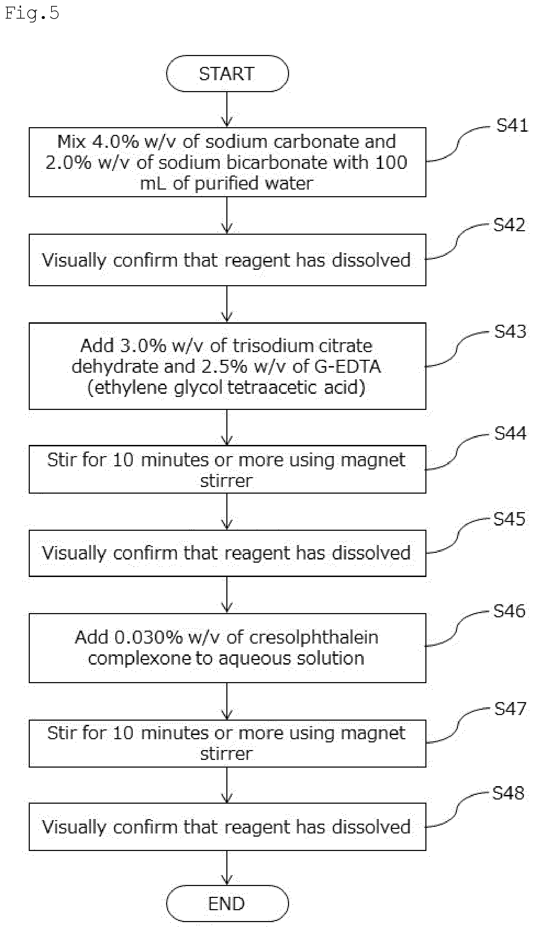

Fig. 5 shows a liquid preparation flow chart of test papers for measuring the concentration of magnesium in urine in Embodiment 5. As shown in Fig. 5, first, 4.0% w/v of sodium carbonate and 2.0% w/v of sodium bicarbonate were mixed with 100 mL of purified water (step S41), and it was visually confirmed that the reagent had dissolved (step S42). Next, 3.0% w/v of trisodium citrate dehydrate and 2.5% w/v of G-EDTA (ethylene glycol tetraacetic acid) were added to this aqueous solution (step S43), and after stirring for 10 minutes or more using a magnet stirrer (SRS311HA manufactured by Toyo Seisakusho Co., Ltd.) (step S44), it was visually confirmed that the reagent had dissolved (step S45). Finally, 0.030% w/v cresolphthalein complexone was added to this aqueous solution (step S46), and after stirring for 10 minutes or more using the magnetic stirrer (step S47), it was visually confirmed that the reagent had dissolved (step S48).

### [Embodiment 6]

### (Method for preparing test papers for measuring the concentration of magnesium in urine)

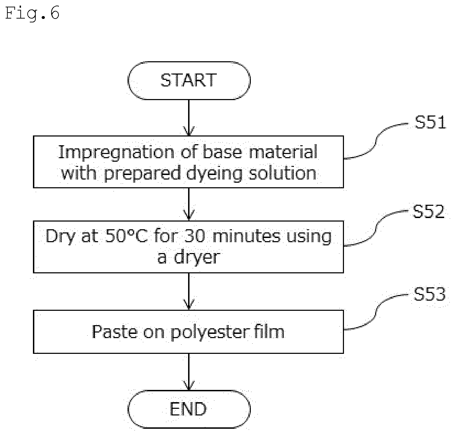

Fig. 6 shows a fabrication flow chart of test papers for measuring the concentration of magnesium in urine. As shown in Fig. 6, the base material (quantitative filter paper No. 3 manufactured by Toyo Roshi Kaisha, Ltd.) was impregnated with the prepared dyeing solution (step S51), and was dried at 50 °C, for 30 minutes using a dryer (DRK432DC manufactured by Toyo Seisakusho Co., Ltd.) (step S52). This was pasted on a polyester film of 85 mm x 5 mm (step S53), and the test paper was completed.

### [Industrial Applicability]

The present invention is useful as test papers for measuring calcium concentration and magnesium concentration in urine.

## Claims

1. A test paper for colorimetrically measuring the concentration of calcium in urine, including:
a coloring agent that colors in response to calcium ions on a substrate,
a magnesium ion sealing agent for preventing coloration due to magnesium ions,
a pH adjustment agent, and
a sensitivity adjustment agent for adjusting the sensitivity of coloration within a range of concentrations of calcium ions in urine.

2. A test paper for colorimetrically measuring the concentration of magnesium in urine, including:
a coloring agent that colors in response to magnesium ions on a substrate,
a calcium ion sealing agent for preventing coloration due to calcium ions,
a pH adjustment agent, and
a sensitivity adjustment agent for adjusting the sensitivity of coloration within a range of concentrations of magnesium ions in urine.

3. The test paper according to claim 1, wherein the coloring agent is cresolphthalein complexone.

4. The test paper according to claim 1 or 3, wherein the magnesium ion sealing agent is 8-Hydroxyquinoline-5-sulfonic acid.

5. The test paper according to claim 2, wherein the coloring agent is cresolphthalein complexone.

6. The test paper according to claim 2 or 5, wherein the calcium ion sealing agent is G-EDTA.

7. The test paper according to any one of claims 1, 3 or 4,

8. The test paper according to any one of claims 1 to 7, wherein the pH adjustment agent is the mixture of sodium carbonate and sodium bicarbonate.

9. The test paper according to any one of claims 1 to 8, wherein the sensitivity adjustment agent is sodium citrate or sodium pyrophosphate.

## Amended claims

### Amended claims under Art. 19.1 PCT

1. A test paper for evaluating nutrition status by colorimetrically measuring the concentration of calcium in urine and the concentration of magnesium in urine, comprising:
a coloring agent that colors in response to calcium ions and magnesium ions on a substrate,
a magnesium ion sealing agent for preventing coloration due to magnesium ions,
a calcium ion sealing agent for preventing coloration due to calcium ions,
a pH adjustment agent, and
a sensitivity adjustment agent for adjusting the sensitivity of coloration within a range of concentrations of calcium ions in urine of 20 to 400 ppm and a range of concentrations of magnesium ions in urine of 18 to 215 ppm.

2. The test paper for evaluating nutrition status according to claim 1, wherein the coloring agent is cresolphthalein complexone.

3. The test paper for evaluating nutrition status according to claim 1 or 2, wherein the magnesium ion sealing agent is 8-Hydroxyquinoline-5-sulfonic acid.

4. The test paper for evaluating nutrition status according to any one of claims 1 to 3, wherein the calcium ion sealing agent is G-EDTA.

5. The test paper for evaluating nutrition status according to any one of claims 1 to 4, wherein the pH adjustment agent is the mixture of sodium carbonate and sodium bicarbonate.

6. The test paper for evaluating nutrition status according to any one of claims 1 to 5, wherein the sensitivity adjustment agent is sodium citrate or sodium pyrophosphate.

7. The test paper for evaluating nutrition status according to any one of claims 1 to 6, wherein the substrate comprises at least one of: paper, plastic, or a nonwoven fabric.

8. The test paper for evaluating nutrition status according to any one of claims 1 to 7, wherein the substrate includes a calcium ion measurement portion and a magnesium ion measurement portion,
wherein the calcium ion measurement portion includes a calcium ion coloring agent that colors in response to calcium ions, a magnesium ion sealing agent that inhibits coloration due to magnesium ions, a calcium pH adjustment agent, and a calcium sensitivity adjustment agent that adjusts a sensitivity of calcium ion measurement portion coloration within a range of concentrations of calcium ions in urine, and
wherein the magnesium ion measurement portion includes a magnesium ion coloring agent that colors in response to magnesium ions, a calcium ion sealing agent that inhibits coloration due to calcium ions, a magnesium pH adjustment agent, and a magnesium sensitivity adjustment agent that adjusts a sensitivity of magnesium ion measurement portion coloration within a range of concentrations of magnesium ions in urine.
